# EUROPÄISCHE PATENTSCHRIFT

(11) **EP 1 129 671 B1**
(45) Veröffentlichungstag und Bekanntmachung des Hinweises auf die Patenterteilung: **06.05.2004**
(21) Anmeldenummer: 01103976.5
(22) Anmeldetag: 19.02.2001
(51) Int. Cl.: A61B 19/00

(54) **Kopfhalterungsvorrichtung für chirurgische Zwecke**
Head clamp
Pince de tête

(30) Priorität: 03.03.2000 DE 10010384
(43) Veröffentlichungstag der Anmeldung: 05.09.2001
(73) Patentinhaber: Zegers, Theo J.J., 5431 NZ Cuijk (NL); Zeppelin von, Kurt Dieter, 82049 Pullach (DE)
(72) Erfinder: Grotenhuis, J.A., 6544 ZZ Nijmegen (NL); Zegers, Theo J.J., 5846 AA Ledeacker (NL); Zeppelin von, Kurt Dieter, 82049 Pullach (DE)
(74) Vertreter: Becker Kurig Straus

(56) Entgegenhaltungen:
- EP-A- 0 776 637
- DE-U- 8 802 938
- GB-A- 2 164 856
- US-A- 5 276 927

## Beschreibung

Die vorliegende Erfindung betrifft eine Kopfhalterungsvorrichtung für chirurgische Zwecke gemäß dem Oberbegriff des Anspruchs 1, insbesondere zur Fixierung des Patientenkopfes intraoperativ in jeder beliebigen, anatomisch möglichen Lage, wobei der Drehpunkt der Kopfhalterung im Halswirbelbereich des Patienten liegt. Eine derartige Kopfhalterungsvorrichtung ist beispielsweise bekannt aus der DE-U 8802938.

Die Präzision chirurgischer Eingriffe im Gehirn, insbesondere in der Neurochirurgie, setzt voraus, daß sich der Kopf des narkotisierten Patienten nicht bewegen darf. Dies wird erreicht mit einer am Operationstisch befestigten Kopfhalterungsvorrichtung.

Aus der US 3,099,441 ist bereits eine Kopfhalterung bekannt, bei der der Kopf zwischen drei Stahldornen geklemmt wird. Die Spitzen dieser Dome sind mit einer einstellbaren Kraft in den Schädel gedrückt. Die Dome befinden sich an zwei diametral gegenüberliegenden Armen. Ein Arm ist mit einem axial verstellbaren Halter für einen Dom und der andere Arm mit einem ebenfalls axial verstellbaren aber mittig verschwenkbaren Halter mit zwei Domen versehen.

Diese Kopfhalterungen haben sich jahrzehntelang in der Praxis bewährt, um den Kopf in der gewünschten und anatomisch richtigen Lage zu fixieren. Mit den bekannten Kopfhalterungen ist es allerdings äußerst schwierig und umständlich, während der Operation die Lage des Kopfes zu verändern. Eine derartige Lageänderung wird jedoch bei minimal invasiven operativen Eingriffen zunehmend notwendig, um das Operationsgebiet zugänglich und sichtbar zu machen.

Ein weiterer Nachteil besteht darin, daß sich die bekannte Anzahl von drei Domen konstruktionsbedingt nur in 1 + 2 aufteilen läßt und daß außerdem die zwei Dome, die sich in einer gemeinsamen meist drehbaren Halterung befinden, axial nicht verstellbar sind, und zwar weder das Maß zwischen den beiden Spitzen noch deren Neigungswinkel. Ein weiterer Nachteil liegt darin, daß sich bestimmte sperrige Teile der Kopfhalterung unter dem Kopf des liegenden Patienten befinden und somit die Beine des sitzend arbeitenden Operateurs oder auch seitlich hinzutretender Assistenten behindern.

Mit der stark zunehmenden Endoskopie in der Neurochirurgie hat die intraoperative Lageveränderung noch an Bedeutung gewonnen. Für Eingriffe mittels eines Endoskops genügt eine viel kleinere Öffnung im Schädel, was die postoperative Wiederherstellung des Patienten erleichtert. Bei endoskopischen und durch Endoskop unterstützten mikroneurochirurgischen Eingriffen ist es entscheidend wichtig, während der Operation die Lage des eingeklemmten Kopfes geringfügig ändern zu können. Dies soll in einfacher Weise, schnell, stufenlos und ergonomisch möglich sein.

Aus der DE 28 09 645 A1 ist bereits ein Gerät für operative Eingriffe am Kopf bekannt, das eine Aufspreizeinrichtung aufweist. Aus der DE-PS 86665 ist bereits ein Schädelhalter bekannt, der eine einfache Halterung des Kopfes ermöglicht.

Aus der DE 198 54 347 A1 ist eine Klemmverbindung für medizinische Geräte und Apparate bekannt, die auch für Kopfhalterungen einsetzbar ist.

Aus der DE-UM 75 24 814 ist eine Haltevorrichtung für medizinische Instrumente bekannt, die einen biegsamen Gliederarm aufweist. Aus der US 4,169,478 ist eine chirurgische Kopfklemme bekannt, die ebenfalls einen Rahmen aufweist. Aus der EP 0 938 873 A2 ist eine stereotaktische Positioniervorrichtung für einen zu bestrahlenden Patienten bekannt.

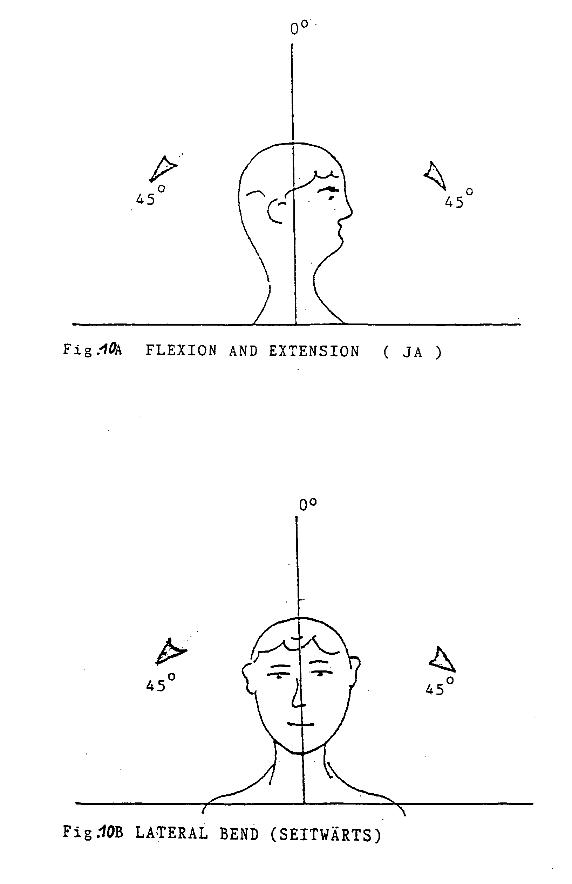

In den Figuren 10A-10D sind die verschiedenen Bewegungsrichtungen des Kopfes am oberen Endpunkt der menschlichen Wirbelsäule erläutert.

Figur 10A zeigt die Flexion und Extension (JA-Bewegung) des Kopfes, bei der der Kopf senkrecht nach vorne und nach hinten geneigt wird. Figur 10B zeigt die Querneigung bzw. seitliche Kippung des Kopfes nach links und rechts.

Figur 10C zeigt die Rotationsbewegung des Kopfes (NEIN-Bewegung).

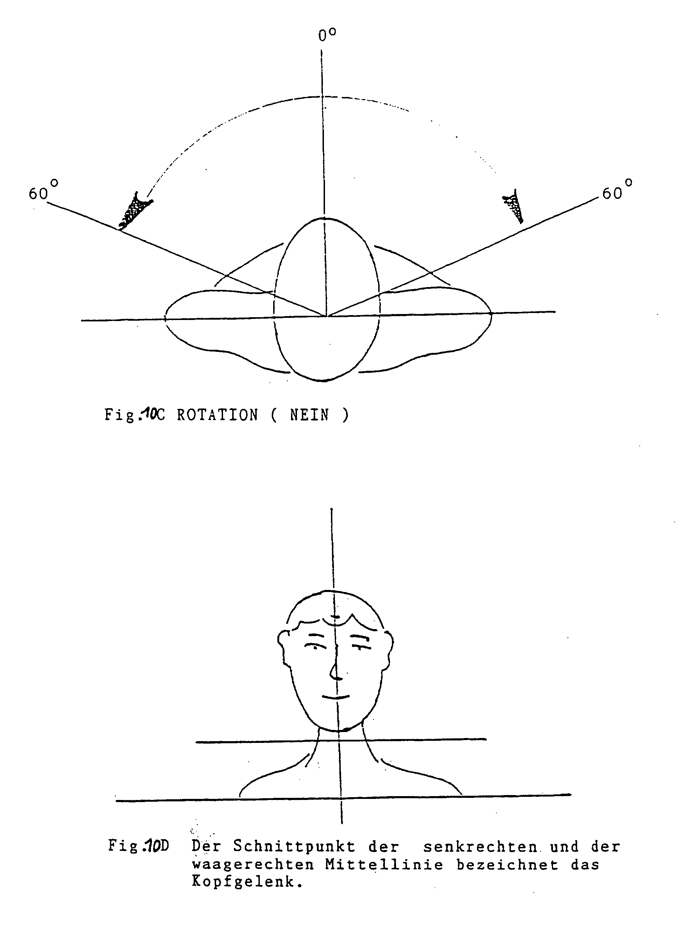

Figur 10D zeigt den Schnittpunkt der senkrechten und waagerechten Mittellinie im Kopfgelenk.

Aus den Figuren 10A-10D geht hervor, daß eine auf die anatomischen Gegebenheiten abgestimmte Kopfhalterung die drei in Figuren 10A-10C gezeigten Freiheitsgrade berücksichtigen muß, während gemäß Figur 10D der Drehpunkt der Kopfhalterung im Halswirbelbereich des Patienten, d. h. bei den beiden oberen Wirbeln (Atlas und Dreher) liegt.

Somit stellen die Neuroendoskopie und die endoskopisch unterstützte Mikroneurochirurgie folgende Anforderungen an eine wirksame Kopfhalterung. Der Kopf sollte in jeder anatomisch möglichen Anfangslage geklemmt und mittels der Kopfhalterung in den drei in Figuren 10A-10C erläuterten Richtungen bewegbar sein. Diese Bewegungen sollten einfach und ohne großen operativen Aufwand möglich sein. Die Bewegungen müssen stufenlos regulierbar und in jeder Lage schnell fixierbar sein. Der Drehpunkt der drei Bewegungen muß in den beiden oberen Halswirbeln liegen. Ferner sollte auch der Drehpunkt der Kopfhalterung in den beiden Mittellinien der besagten Halswirbel des Patienten liegen. Der Raum unterhalb des Kopfes des liegenden Patienten sollte möglichst frei von behindernder Mechanik sein.

Der Erfindung liegt somit die Aufgabe zugrunde, eine Kopfhalterungsvorrichtung für chirurgische Zwecke zu schaffen, welche die genannten Bedingungen erfüllt und außerdem eine Aufteilung der mindestens zwei Dome in 1 + 1 ermöglicht, also mindestens zwei Halterungen mit jeweils einem Dom ermöglicht, wobei die zwei oder auch drei Halterungen zueinander stufenlos verstellbar sind, und zwar derart, daß der Schnittpunkt der Verlängerungslinien der drei Dome immer in der senkrechten Mittellinie der oberen Halswirbel liegt.

Diese Aufgabe wird erfindungsgemäß gelöst durch eine Kopfhalterungsvorrichtung gemäß Anspruch 1. Der Rahmen läßt sich um eine Achse (x) schwenken, die somit die in Figur 10B gezeigte seitwärtige Kippbewegung wiedergibt. Das um eine Achse (y) drehbar gelagerte Profil ermöglicht die in Figur 10A gezeigte Vorder- und Rückneigung des Kopfes (JA-Bewegung). Schließlich ermöglicht das um eine Achse (z) schwenkbare Gegenprofil die in Figur 10C gezeigte Drehbewegung (NEIN-Bewegung) des Kopfes.

Es ist bevorzugt, daß das Profil und das Gegenprofil durchgehend kreissegmentförmig gekrümmt sind. Damit verschiebt sich der Mittelpunkt bei Verschwenkung des Gegenprofils um das Profil nicht.

Bevorzugt ist eine Kopfstütze an dem Gegenprofil befestigbar. Damit kann eine zusätzliche Abstützung des Kopfes bei Verwendung von zwei Haltedornen erzielt werden.

Die Kopfhalter sind an beliebiger Position an dem Gegenprofil befestigbar. Dadurch lassen sich die Halterungspunkte frei verstellen und wieder arretieren.

Die Schwenkbewegungen um die Achsen (x, y, z) sind stufenlos möglich und in jeder Position fixierbar bzw. selbsthemmend ausgestaltet. Damit ist die Kopfhalterungsvorrichtung universell einsetzbar und bequem neu positionierbar.

Bevorzugt stehen die Achsen (x, y, z) senkrecht aufeinander, so daß mit drei voneinander unabhängigen Freiheitsgraden jede Kopfposition im Rahmen des verfügbaren Kopfneigungswinkels einstellbar ist.

Weitere Vorteile, Merkmale und Anwendungsmöglichkeiten der vorliegenden Erfindung ergeben sich aus der nachfolgenden Beschreibung eines Ausführungsbeispiels in Verbindung mit der Zeichnung.

Fig. 1 zeigt eine Ausführungsform der erfindungsgemäßen Kopfhalterungsvorrichtung in Vorderansicht.

Fig. 2 zeigt die erfindungsgemäße Ausführungsform der Kopfhalterung in Seitenansicht.

Fig. 3 zeigt ein Gegenprofil zur Verwendung mit der Kopfhalterungsvorrichtung gemäß der Erfindung.

Fig. 4 zeigt ein Profil zur Verwendung in der Kopfhalterungsvorrichtung gemäß der Erfindung.

Fig. 5 zeigt einen Kopfhalter zur Verwendung mit der Kopfhalterungsvorrichtung gemäß der Erfindung.

Fig. 6 zeigt einen Haltedorn zur Verwendung in der Kopfhalterungsvorrichtung gemäß der Erfindung.

Fig. 6a zeigt eine alternative Ausführungsform eines Kopfhalters zur Verwendung in der Kopfhalterungsvorrichtung gemäß der Erfindung.

Fig. 7 zeigt einen Rahmen zur Verwendung in der Kopfhalterungsvorrichtung gemäß der Erfindung.

Fig. 8 zeigt die Befestigung einer Kopfhalterungsvorrichtung gemäß der Erfindung an einem Operationstisch in Vorder- und Seitenansicht.

Fig. 9 zeigt eine Kopfstütze zur Verwendung in der Kopfhalterungsvorrichtung gemäß der Erfindung in Aufsicht und Schnittansicht.

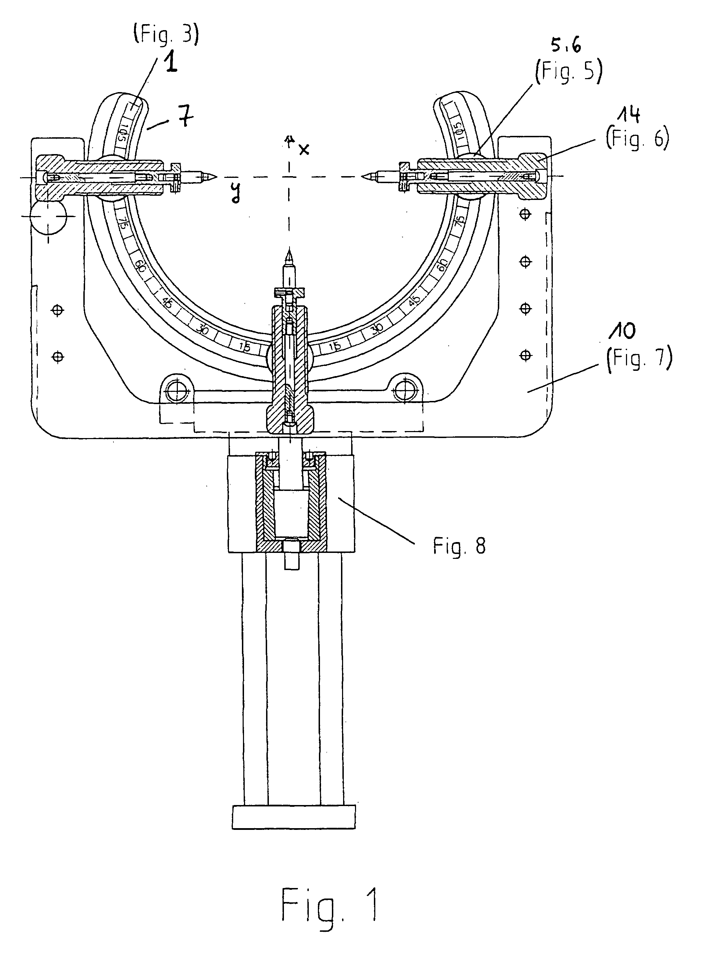

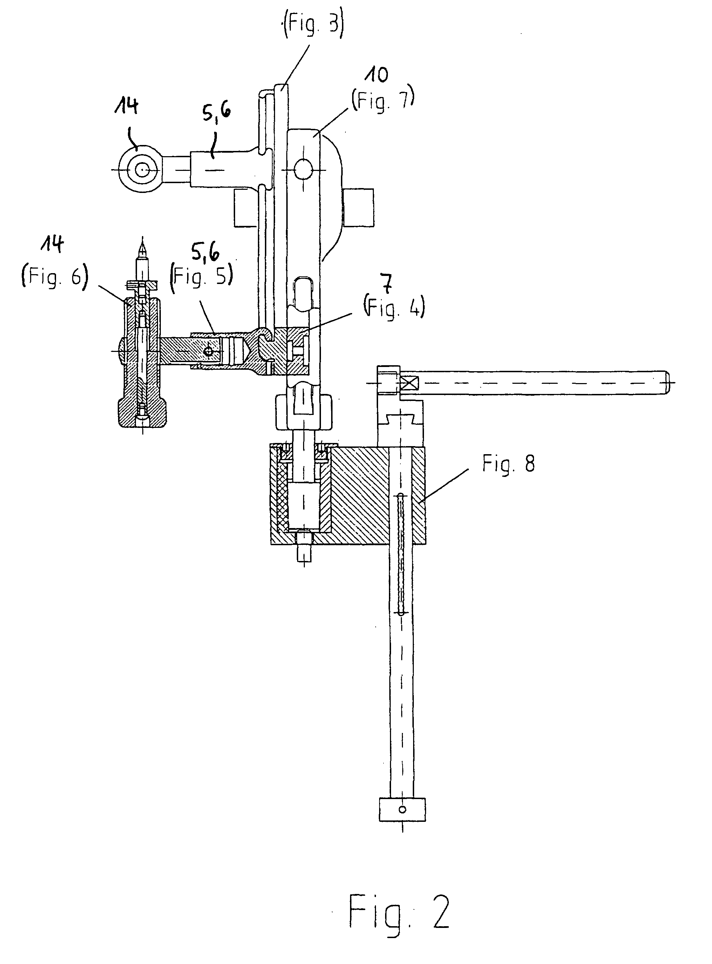

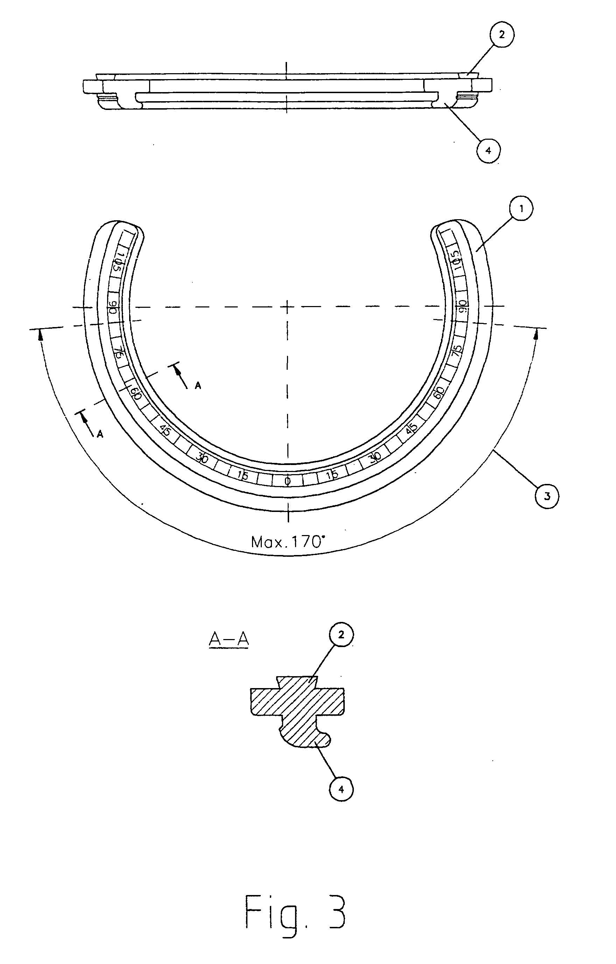

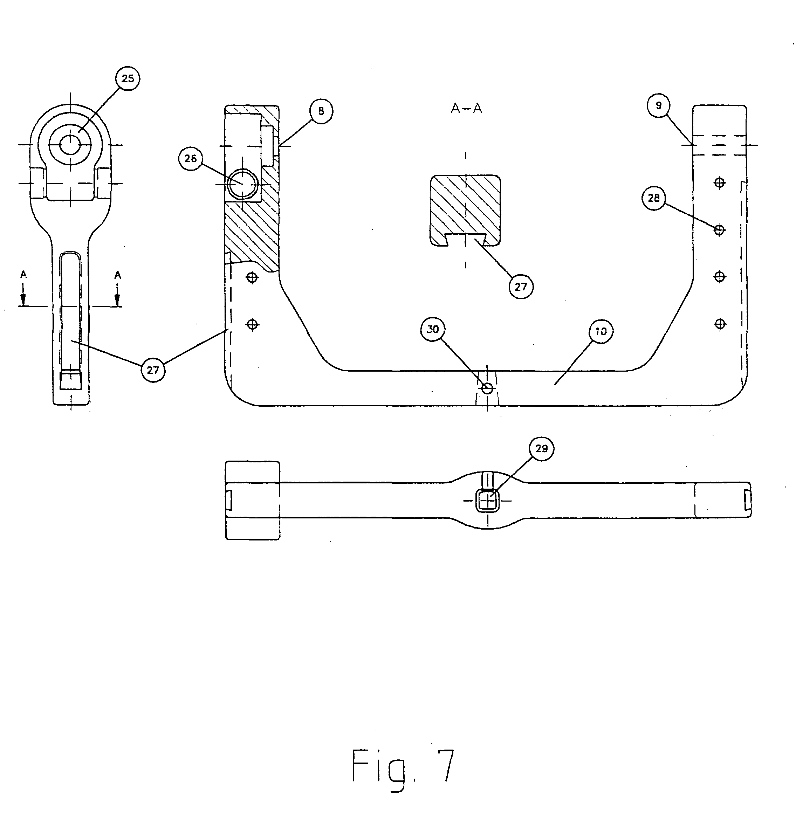

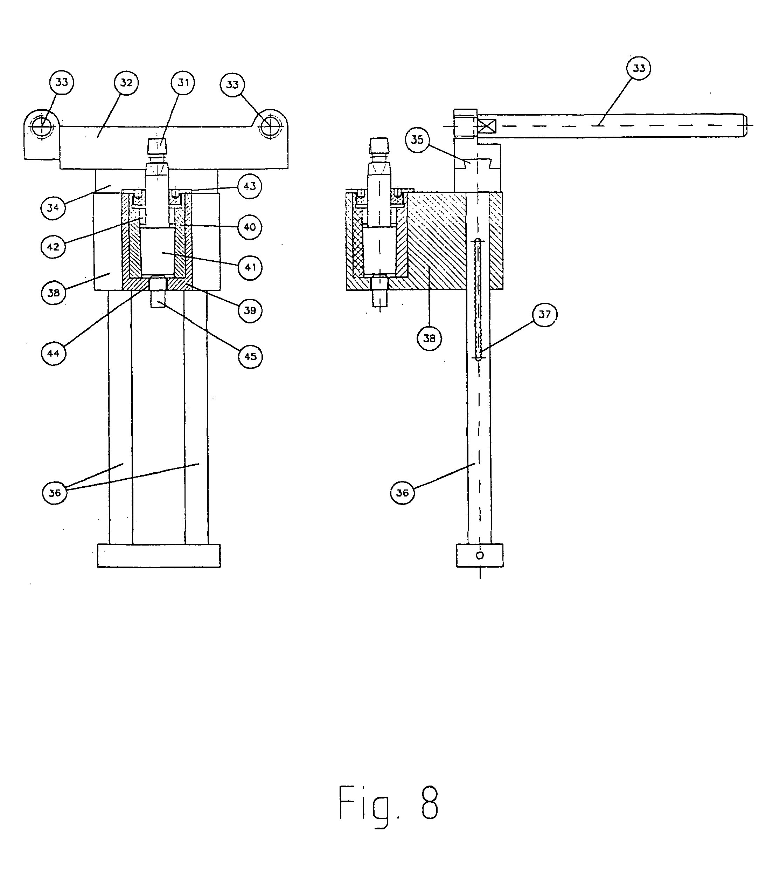

In den Figuren 1 und 2 sind in Vorder- und Seitenansicht alle Elemente der Kopfhalterungsvorrichtung gemäß der Erfindung deutlich gezeigt. Ein mit zwei gegenüberliegenden Schenkeln ausgestatteter Rahmen 10, wie in Fig. 7 erläutert, ist um eine Achse (x) schwenkbar gelagert und an einem Operationstisch, wie er in Fig. 8 erläutert ist, befestigt. An dem Rahmen 10, d. h. zwischen den gegenüberliegenden Schenkeln ist ein um eine Achse (y) schwenkbares Profil 7 befestigt, das mit einem Gegenprofil 1, wie es in Fig. 3 erläutert ist, zusammenwirkt, um um eine Achse (z) schwenkbar zu sein. An dem Gegenprofil 1 sind einander gegenüberliegend zwei Kopfhalter 5, 6 befestigt, die jeweils einen Haltedom 14 tragen. Ein weiterer Kopfhalter ist an der unterst möglichen Position des Gegenprofils 1 befestigt.

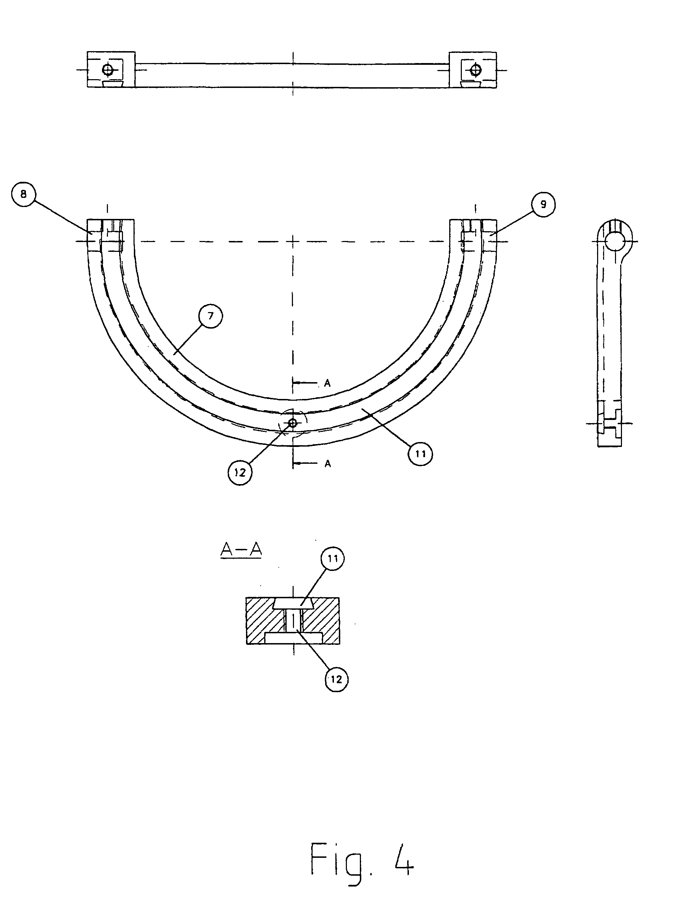

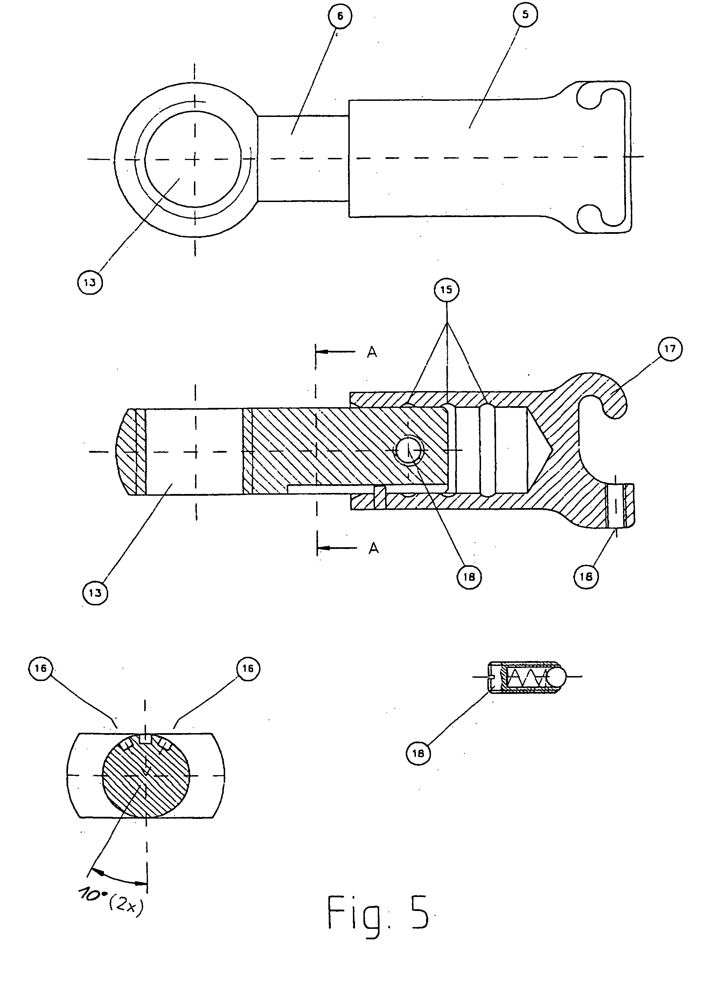

Fig. 3 zeigt das schwenkbare, profilierte und mit einer Gradeinteilung versehene kreissegmentförmige Gegenprofil 1, das für die NEIN-Bewegung (Rotation) ausgebildet ist. Das im Querschnitt hintere Profilteil 2 stellt den männlichen Teil einer Schwalbenschwanzführung mit einem kreissegmentförmigen Bogenmaß 3 von 170° dar. Der hintere Profilteil 2 ist somit zum Eingriff in dem in Fig. 4 gezeigten Profil 7 bestimmt. Der vordere Profilteil 4 ist mit einer im Schnitt gezeigten Schulter versehen und für das Einrasten der axial verstellbaren zweiteiligen Kopfhalter 5, 6, 14 bestimmt, wie sie in Fig. 5 gezeigt sind. Somit ist der hintere Profilteil 2 ein vorspringender schwalbenschwanzförmiger Eingriffsteil, während der vordere Profilteil 4 ein mit einer Schulter versehener vorspringender Teil ist.

Fig. 4 zeigt ein profiliertes kreissegmentförmiges Profil 7 für die JA-Bewegung (Flexion, Extension) in Seitenansicht, Vorderansicht, Aufsicht und im Schnitt. Das Profil 7 hat zwei Ausnehmungen bzw. Bohrungen 8, 9, mit denen es an zwei Achswellen schwenkbar im Rahmen 10 (Fig. 7) gelagert ist. Das Profil 7 hat einen hinteren Teil und einen vorderen Teil 11, der der weibliche Teil einer Schwalbenschwanzführung ist, der zum Eingriff des Profilteils 2 des Gegenprofils 1 bestimmt ist. In einer Gewindebohrung 12 läßt sich das Gegenprofil 1 mit einer handelsüblichen Klemmspannschraube kraftschlüssig im Profil 7 fixieren.

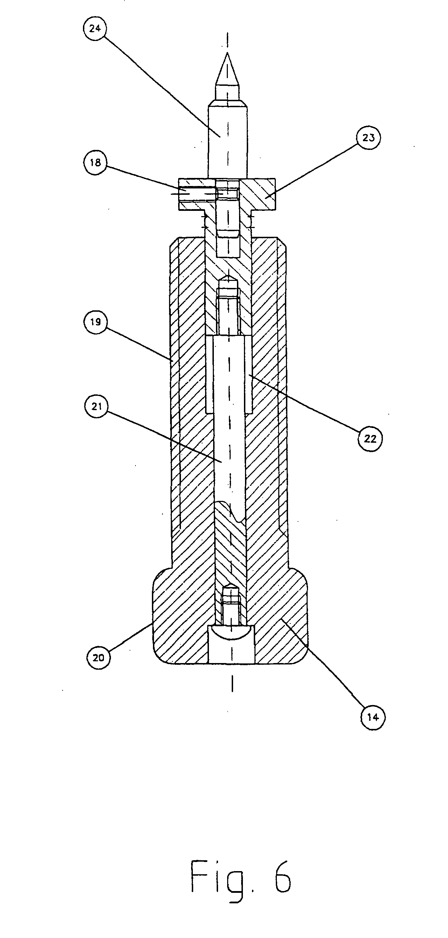

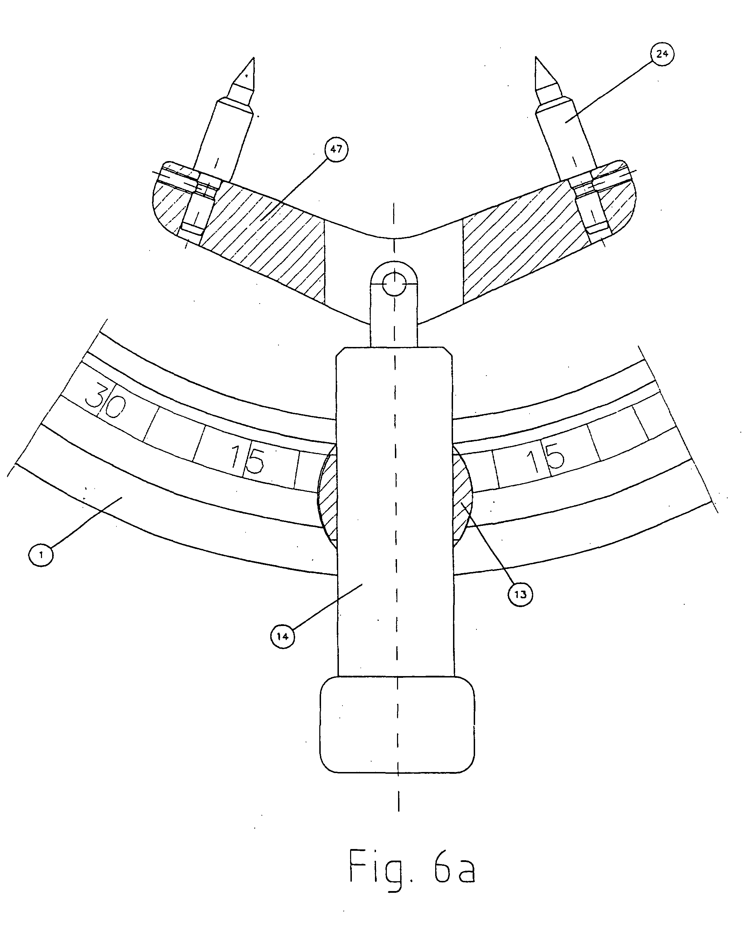

Fig. 5 zeigt verschiedene Ansichten eines axial verstellbaren zweiteiligen Kopfhalters 5, 6. Eine Gewindebohrung 13 dient zur Aufnahme eines radial verstellbaren Haltedoms 14 (Fig. 6). Der Haltedorn 14 läßt sich in drei verschiedenen Positionen axial positionieren, und zwar -90° und 10° nach links und rechts. In jedem möglichen Stand läßt sich der Kopfhalter mittels eines handelsüblichen federnden Druckstücks 18 durch Einrasten fixieren. Der vordere Teil 5 des Kopfhalters ist vorne bei (17) nockenartig ausgebildet, und zwar derart, daß er in den vorderen Teil (4) des Gegenprofils 1 (NEIN-Bewegung) eingerastet werden kann und mittels eines handelsüblichen federnden Druckstücks (18) in dieses Profil einrastet. Die Befestigung des Einrastens des Kopfhalters 5, 6 in dem Profilteil 4 macht ferner ein Verschieben in beide Richtungen und somit eine variable Position des Kopfhalters im Gegenprofil möglich.

Fig. 6 zeigt im Schnitt einen Haltedom 14, der radial verstellbar ist. Der Haltedorn besteht aus einer Schraube 19 mit angedrehtem Handrad 20. Diese Kombination 19, 20 ist mit einer Bohrung versehen zur Aufnahme einer Stange 21, einer Druckfeder 22, einer Dornaufnahme 23 und eines Doms 24. Der Dom 24 wird in der Aufnahme 23 mittels eines handelsüblichen federnden Druckstücks 18 gehalten.

Fig. 6a zeigt eine Ausführungsform des Kopfhalters mit zwei Dornen. Zwei Dome 24 sind an einander gegenüberliegenden Positionen bevorzugt symmetrisch in einer gemeinsamen sowohl drehbaren als auch schwenkbaren Halterung 47 befestigt. Dieser alternative Haltedorn läßt sich ebenfalls in die Gewindebohrung 13 des axial verstellbaren Kopfhalters 5, 6 einschrauben und somit in das vordere Teil 4 des Gegenprofils 1 für die NEIN-Bewegung einrasten. Somit läßt sich auch der alternative Kopfhalter in derselben Befestigungsart wie zu Figur 5 beschrieben einrasten.

Fig. 7 zeigt einen Rahmen 10 in Aufsicht, Seitenansicht und Bodenansicht sowie im Schnitt. Die beiden gegenüber liegenden Schenkel sind deutlich erkennbar. Es sind Bohrungen 8, 9 gebildet, die zur Aufnahme des Profils 7 (Fig. 4) dienen. Das Profil 7 ist mittels zwei Wellen in den Bohrungen 8, 9 im Rahmen drehbar gelagert und wird mittels eines Schneckengetriebes 25 mit Drehknopf 26 bewegt. Die Selbsthemmung des Schneckengetriebes 25 bewirkt eine formschlüssige Fixierung des Profils 7 in jeder Position. Der Rahmen 10 ist an beiden Stirnseiten der Schenkel mit dem weiblichen Teil einer Schwalbenschwanzführung 27 sowie mit einer Anzahl Bohrungen 28 versehen. Die Schwalbenschwanzführung dient ebenso wie die Bohrungen 28 der Befestigung von Hilfseinrichtungen für die Operation. Mit einer konischen Vierkantbohrung 29 im die beiden Schenkel verbindenden Horizontalbalken des Rahmens 10 wird dieser auf einen externen Träger, beispielsweise eine Befestigungsvorrichtung 31 (Fig. 8) aufgesteckt und mittels eines handelsüblichen federnden Rastbolzens in einer Gewindebohrung 30 verriegelt.

Fig. 8 zeigt die Montage der Kopfhalterungsvorrichtung gemäß der Erfindung an einer Befestigungseinrichtung 31 eines Operationstisches. Diese Einrichtung besteht aus einer radial verstellbaren zweiteiligen Schiene 32 mit zwei Befestigungsstangen 33 zum Einstecken in die Rohre, mit denen jeder neurochirurgische OP-Tisch ausgerüstet ist, einer zweiten Schiene 34, die in der ersten Schiene 32 in einer Schwalbenschwanzführung 35 verstellbar und mit zwei Bohrungen zur Aufnahme von zwei Führungsstangen 36 versehen ist. In der mittigen Gewindebohrung werden beide Schienen 32, 34 mittels einer handelsüblichen Klemmspannschraube fixiert.

Eine der beiden Führungsstangen 36 ist mit einer Vielzahl von aneinander gereihten Bohrungen 37 versehen derart, daß die nächste Bohrung die vorangehende schneidet. In dieser Aneinanderreihung von Bohrungen kann der komplette Rahmen 10 mit einem handelsüblichen Rastbolzen in der gewünschten Höhe fixiert werden.

Der Träger 38 gleitet über die Führungsstangen 36 des Rahmens 10. Dieser Träger 38 besteht aus einem Gehäuse 39, einer Büchse mit Kegelhülse 40, einer Welle mit Kegelschaft 41 und konischem Vierkant, einer Druckfeder 42 und einem Deckel 43. Der Gehäuseboden hat eine Gewindebohrung 44 zur Aufnahme eines Gewindebolzens 45, der mit einer handelsüblichen Klemmnabe mit Griffstange versehen ist. Durch Drehen der Klemmnabe mittels der Griffstange bewegt sich der Bolzen um einen Teil seiner Gangsteigung nach oben und löst die Selbsthemmung des Kegels. Dadurch wird der Rahmen 10 drehbar und die laterale Neigung des Kopfes des Patienten einstellbar.

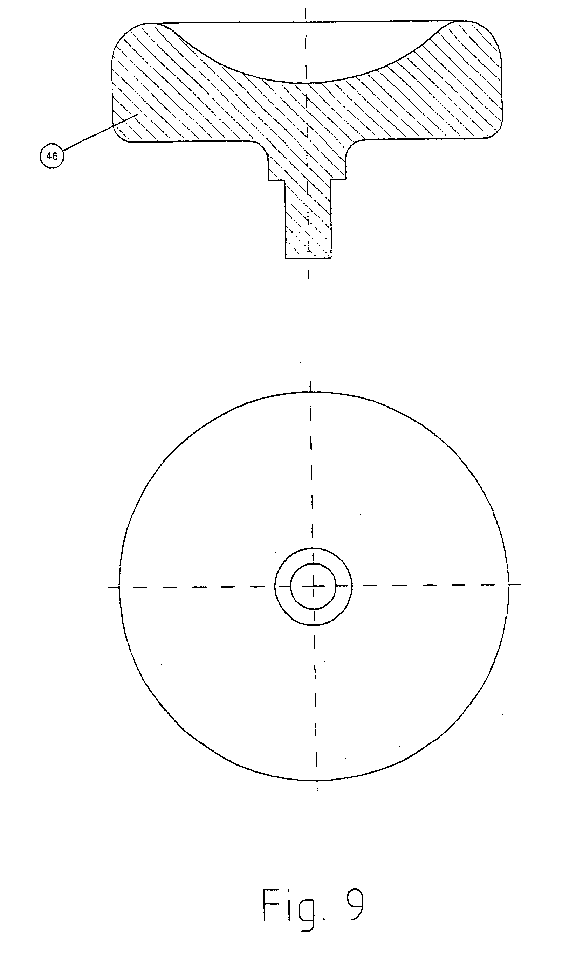

Fig. 9 zeigt eine der Schädelform angepaßte Kopfstütze 46 in Aufsicht und im Schnitt. Diese Kopfstütze ist einsteckbar in einen Kopfhalter mit angedrehtem Handrad 19, 20 und erleichtert das Einklemmen des Kopfes mittels der Dorne 24. Die Kopfstütze 46 ermöglicht außerdem intraoperative Röntgenaufnahmen dadurch, daß der nicht-röntgendurchlässige Kopfhalter mit den Domen durch einfaches Ausrasten entfernt und nach den Aufnahmen wieder angebracht werden kann.

Die Erfindung liefert somit eine Kopfhalterungsvorrichtung zum Klemmen und Fixieren des Kopfes eines Patienten, insbesondere für neurochirurgische Eingriffe, bei der der Kopf in jeder anatomisch möglichen Position geklemmt und fixiert werden kann und von dieser Position aus der Kopf mittels der Halterung in drei Richtungen, nämlich vor und zurück, seitlich geneigt, gedreht, bewegbar ist.

Die erfindungsgemäße Kopfhalterung zeichnet sich dadurch aus, daß der Drehpunkt der Halterung für jede der vorgenannten Bewegungen in der Mittellinie der Halwirbel des Patienten liegt. Die genannten Bewegungen sind mittels der erfindungsgemäßen Kopfhalterungsvorrichtung stufenlos verstellbar und in jeder Position entweder mittels einer Arretiervorrichtung fixierbar oder selbsthemmend.

Bei der erfindungsgemäßen Kopfhalterungsvorrichtung lagern die mit Dornen versehenen Halterungen in einem vorzugsweise zweiteiligen Kreissegment, welches mittels zwei seitlich angebrachter Wellen im Rahmen schwenkbar gelagert und entweder fixierbar oder selbsthemmend ist, um die JA-Bewegung des Kopfes zu ermöglichen.

Die ineinander gelagerten Kreissegmente sind bei der erfindungsgemäßen Kopfhalterung gegeneinander verdrehbar und fixierbar, um eine NEIN-Bewegung (Rotation) des Kopfes zu ermöglichen.

Bei der erfindungsgemäßen Kopfhalterungsvorrichtung ist der vordere Teil des Kreissegmentes bei der NEIN-Bewegung (Rotation) derart profiliert, daß die axial verstellbaren Kopfhalter mit den eingeschraubten radial verstellbaren Haltedornen an beliebiger Stelle eingerastet werden können und von dort aus in beiden Richtungen verschieblich sind.

Das Kreissegment für die NEIN-Bewegung (Rotation) ist vom unteren Nullpunkt aus nach beiden Richtungen gleichmäßig mit einer Gradeinteilung versehen, um eine gradgenaue Positionierung gewährleisten zu können.

Der am Befestigungsteil des Operationstisches angebrachte Rahmen ist bei der erfindungsgemäßen Kopfhalterungsvorrichtung schwenkbar und in jeder Position fixierbar, um die Seitwärtskippbewegung (Fig. 10B) des Kopfes zu ermöglichen. Der am Befestigungsteil des Operationstisches angebrachte Rahmen ist nahezu stufenlos höhenverstellbar und vorzugsweise mit einem handelsüblichen Rastbolzen in jeder Position des Verstellbereiches fixierbar.

Mit dem radial und axial verstellbaren Kopfhalter kann sowohl der Dorn als auch eine der Schädelform angepaßte Kopfstütze eingesetzt werden, und zwar in der gleichen Befestigungsart mittels eines handelsüblichen federnden Druckstückes.

Die erfindungsgemäße Kopfhalterungsvorrichtung ist mittels der vorgenannten Befestigungseinrichtungen sowohl bei operativen Eingriffen am liegenden als auch am sitzenden Patienten verwendbar und liefert damit eine vielseitige und unterschiedlichen Gegebenheiten anpaßbare Kopfhalterung.

## Patentansprüche

1. Kopfhalterungsvorrichtung für chirurgische Zwecke, aufweisend
einen an einem externen Träger befestigbaren Rahmen (10), der um eine Achse (x) schwenkbar ist, wobei der Rahmen einander gegenüberliegende Schenkel aufweist,
**gekennzeichnet durch**
ein zwischen den Schenkeln angeordnetes, an den Schenkeln befestigtes und um eine Achse (y) drehbar gelagertes Profil (7), wobei das Profil (7) zumindest abschnittsweise gleichmäßig gekrümmt ist und in dem gekrümmten Bereich eine Schiene (11) aufweist,
ein in der Schiene (11) des Profils (7) verschieblich gelagertes Gegenprofil (1), wobei das Gegenprofil (1) bei Verschiebung um eine Achse (z) schwenkbar ist, und
zumindest zwei an dem Gegenprofil (1) befestigbare Kopfhalter (5, 6, 14), die Haltedome (14) aufweisen, zwischen denen der Kopf gehalten und fixiert wird.

2. Kopfhalterungsvorrichtung nach Anspruch 1, **dadurch gekennzeichnet, daß** das Profil (7) und das Gegenprofil (1) durchgehend kreissegmentförmig gekrümmt sind.

3. Kopfhalterungsvorrichtung nach Anspruch 1 oder 2, **dadurch gekennzeichnet, daß** eine Kopfstütze (46) an dem Gegenprofil (1) befestigbar ist.

4. Kopfhalterungsvorrichtung nach einem der vorhergehenden Ansprüche, **dadurch gekennzeichnet, daß** die Kopfhalter (5, 6, 14) an beliebiger Position an dem Gegenprofil (1) befestigbar sind.

5. Kopfhalterungsvorrichtung nach einem der vorhergehenden Ansprüche, **dadurch gekennzeichnet, daß** die Schwenkbewegungen um die Achsen (x, y, z) stufenlos möglich und in jeder Position fixierbar bzw. selbsthemmend sind.

6. Kopfhalterungsvorrichtung nach einem der vorhergehenden Ansprüche, **dadurch gekennzeichnet, daß** die Achsen (x, y, z) senkrecht aufeinander stehen.

## Claims

1. Head holding device for surgical purposes, comprising
a frame (10) which is mountable on an external support, and which is pivotable around an axis (x), wherein said frame comprises legs facing each other,
**characterized by**
a profile (7) being located between said legs and mounted on said legs and supported rotatably around an axis (y), wherein said profile (7) is uniformly arcuate at least in sections thereof and comprises a rail (11) in said arcuate area,
a counterprofile (1) which is displaceably supported in said rail (11) of said profile (7), wherein said counterprofile (1) is pivotable around an axis (z) upon displacement, and
at least two head holders (5, 6, 14) which are mountable on said counterprofile (1), and which comprise holding mandrels (14) between which the head ist held and fixed.

2. Head holding device according to claim 1, **characterized in that** said profile (7) and said counterprofile (1) are continously arcuate in the shape of a circular segment.

3. Head holding device according to claim 1 or 2, **characterized in that** a headrest (46) is mountable on said counterprofile (1).

4. Head holding device according to anyone of the preceding claims, **characterized in that** said head holders (5, 6, 14) are mountable on said counterprofile (1) in any position.

5. Head holding device according to anyone of the preceding claims, **characterized in that** the pivoting movements around said axes (x, y, z) are countinously possible and are fixeable or self-locking, respectively, in each position.

6. Head holding device according to anyone of the preceding claims, **characterized in that** said axes (x, y, z) are perpendicular to each other.

## Revendications

1. Dispositif de têtière de fixation à usages chirurgicaux, présentant
un cadre (10) pouvant être fixé à un support externe, lequel cadre peut pivoter autour d'un axe (x), moyennant quoi le cadre présente des montants opposés les uns aux autres,
**caractérisé en ce que**
un profil (7) disposé entre les montants, fixé à ces derniers et pivotant autour d'un axe (y), moyennant quoi le profil (7) est courbé de manière uniforme au moins par sections et présente un rail (11) dans la zone courbée,
un contre-profil (1) disposé de manière déplaçable dans le rail (11) du profil (7), moyennant quoi le contre-profil (1) peut pivoter autour d'un axe (z) en cas de déplacement, et
au moins deux têtières de fixation (5, 6, 14) pouvant être fixées au contre-profil (1), qui présentent des goujons de maintien, entre lesquels la tête est maintenue et fixée.

2. Dispositif de têtière de fixation selon la revendication 1, **caractérisé en ce que** le profil (7) et le contre-profil (1) sont courbés, sur toute la longueur, en forme de segment de cercle.

3. Dispositif de têtière de fixation selon la revendication 1 ou 2, **caractérisé en ce qu'**un repose-tête (46) peut être fixé au contre-profil (1).

4. Dispositif de têtière de fixation selon l'une quelconque des revendications précédentes, **caractérisé en ce que** les têtières de fixation (5, 6, 14) peuvent être fixées à une quelconque position sur le contre-profil (1).

5. Dispositif de têtière de fixation selon l'une quelconque des revendications précédentes, **caractérisé en ce que** les mouvements pivotants autour des axes (x, y, z) sont possibles en continu et sont fixables ou autobloquants dans chaque position.

6. Dispositif de têtière de fixation selon l'une quelconque des revendications précédentes, **caractérisé en ce que** les axes (x, y, z) sont situés les uns sur les autres verticalement.
